# FASCICULE DE BREVET EUROPEEN

(11) **EP 0 640 124 B1**
(45) Date de publication et mention de la délivrance du brevet: **02.10.1996**
(21) Numéro de dépôt: 93910090.5
(22) Date de dépôt: 06.05.1993
(51) Int. Cl.: C12M 1/40, C12M 3/06, C12M 1/12

(54) **DISPOSITIF DU TYPE REACTEUR A VOLUME VARIABLE ET PROCEDE DE CULTURE DE MATERIAL CELLULAIRE**
REAKTOR MIT VERAENDERLICHEM VOLUMEN UND ZELLKULTURVERFAHREN
VARIABLE VOLUME REACTOR-TYPE DEVICE AND PROCESS FOR THE CULTURE OF CELLULAR MATERIAL

(30) Priorité: 06.05.1992 FR 9205579
(43) Date de publication de la demande: 01.03.1995
(73) Titulaire: L.V.M.H. RECHERCHE, F-92700 Colombes (FR)
(72) Inventeur: BOULAY, Michel, F-7700 Melun (FR); DELOIRE, Alain, F-51100 Reims (FR); MAURO, Marie-Claude, F-51270 Orbais l'Abbaye (FR); MEYBECK, Alain, F-92400 Courbevoie (FR); PIERRE, Guy, F-31500 Toulouse (FR); RABAUD, Jean-No[l, F-31400 Toulouse (FR)
(74) Mandataire: Warcoin, Jacques
(86) Numéro de dépôt international: FR9300437
(87) Numéro de publication internationale: WO9322420

(56) Documents cités:
- EP-A- 0 269 086
- EP-A- 0 322 749
- EP-A- 0 428 800
- EP-A- 0 473 011
- DE-A- 3 327 541
- FR-A- 1 589 364
- FR-A- 2 507 496
- FR-A- 2 519 651
- CHEMIKER-ZEITUNG, vol. 108, no. 5, May 1984, Heidelberg (DE); E.A. STADLBAUER et al, pp. 165-176

## Description

La présente invention concerne un dispositif de culture de matériel biologique cellulaire sous forme de particules solides en présence d'un milieu de culture liquide. On entend ici par "particules solides de matériel biologique cellulaire" des particules constituées, en tout ou en partie de matière biologique cellulaire, l'autre partie le cas échéant étant constituée de matière inerte comme support ou matériau d'immobilisation. Il peut s'agir, par exemple, d'agrégats cellulaires ou de parties de tissus végétaux tels que des morceaux de racines, ou encore des cellules isolées revêtues sur des supports inertes. Lesdites particules étant généralement fines, bien que non nécessairement, le dispositif de l'invention peut en effet constituer un appareil de culture cellulaire en milieu liquide, lesdites particules comprenant alors des cellules. Ce dispositif comporte une enceinte destinée à se remplir complètement dudit liquide et à contenir lesdites particules solides en contact avec le liquide. Il comprend ainsi des moyens d'alimentation et d'évacuation du liquide de l'enceinte et des moyens de retenue desdites particules solides et séparation d'avec le liquide pour maintenir celles-ci à l'intérieur de l'enceinte dans une zone déterminée et éventuellement des moyens de circulation et traitement du liquide en dehors de l'enceinte. La présente invention a également pour objet un procédé pour réaliser la réaction des particules avec le liquide, notamment un procédé de culture de cellules en milieu liquide à l'aide d'un tel dispositif.

Un réacteur de ce type a été décrit dans la demande de brevet internationale WO 86 05202 et dans DE-A-3327541. Toutefois, le dispositif décrit antérieurement ne disposait pas d'un volume variable. Or précisément, dans certains cas, tel que la culture d'embryons somatiques de végétaux, il apparaît qu'il peut être avantageux, voire nécessaire, d'effectuer une culture à densité volumique constante de cellules pour avoir un développement correct des embryons ou même de diminuer cette densité par l'augmentation du volume de l'enceinte de culture, en partie à cause du relargage de certains composés lors de la maturation des embryons, ces composés pouvant avoir un effet stimulateur ou inhibiteur de la maturation.

Par ailleurs, le réacteur décrit dans les demandes de brevet précitéés comporte, à proximité de la zone de culture, une hélice (28) destinée à créer une circulation du milieu liquide, de telle sorte qu'elle maintienne les particules solides ou cellules en régime de lit fluidisé. Or, cette disposition comporte le risque de blessure ou d'altération des particules ou des cellules, en particulier si celles-ci s'échappent du lit fluidisé, par exemple en cas de dérèglement ou de dysfonctionnement du système.

EP-A-0 428 800 décrit un bioréacteur permettant de réaliser des réactions enzymatique comprenant une paroi mobile constituée par une vis qui permet d'ajuster et de maintenir constant le volume réactionnel. EP-A-473 011 décrit une colonne de préparation d'enzymes à partir de bactéries, la colonne étant munie d'un piston pour éluer les enzymes produites.

Un but de la présente invention est de fournir un bio-réacteur qui permette d'effectuer une culture de matériel biologique cellulaire à densité volumique cellulaire constante au fur et à mesure du développement de la culture et, en outre, pallie aux inconvénients des réacteurs précités.

Pour ce faire, la présente invention fournit un dispositif de culture de matériel biologique cellulaire sous forme de particules solides dans lequel on met en contact les dites particules solides avec un milieu de culture liquide, permettant de maintenir sensiblement constante la densité volumique du dit matériel biologique cellulaire par rapport au volume du dit milieu de culture, comportant :
- une chambre de culture constituée d'une enceinte (5) délimitée par des parois, dont au moins l'une des dites parois (I) peut être mobile de sorte que l'enceinte a un volume variable.
- des moyens (3a et 3b) d'alimentation, d'évacuation et de circulation du liquide dans et de l'enceinte, et
- des moyens (1a, 6) de retenue des dites particules et séparation d'avec le liquide pour maintenir celles-ci à l'intérieur de l'enceinte,
caractérisé en ce qu'il comporte des moyens pour faire varier le volume du milieu de culture dans la chambre de culture, notamment pour faire augmenter le volume du milieu de culture au fur et à mesure du développement de la culture et de l'augmentation du volume de matériel biologique cellulaire, constitués par au moins l'une des dites parois (1) mobiles de l'enceinte.

Dans un mode de réalisation du dispositif selon l'invention l'enceinte définit, en partie au moins, un cylindre, ladite paroi mobile de l'enceinte étant constituée par une base du cylindre.

En particulier, l'enceinte définit en partie au moins un cylindre qui coopère de façon étanche avec un piston lequel peut être déplacé à l'intérieur du cylindre de manière à faire varier le volume de l'enceinte en fonction de sa position, la base du piston constituant une paroi mobile de l'enceinte.

De façon appropriée, l'enceinte est constituée d'une colonne cylindrique, les moyens d'alimentation et évacuation du liquide étant situés à chaque extrémité de celle-ci, l'une de ces extrémités étant constituée par ledit cylindre et ledit piston, et le piston incluant un moyen d'alimentation ou d'évacuation du liquide de l'enceinte à l'une des extrémités de la colonne, un autre moyen d'alimentation ou d'évacuation du liquide étant situé à l'autre extrémité.

Dans un mode de réalisation particulièrement utile, les moyens précités de retenue des particules et de séparation d'avec les liquides peuvent consister dans deux grilles dont la maille ne laisse pas passer les particules solides, ces moyens délimitant, avec les autres parois de l'enceinte, une zone de contact entre liquide et particules solides,

Avantageusement, le maillage des grilles sera choisi notamment en fonction de l'un ou plusieurs des paramètres suivants, accessibles à l'homme de l'art : dimensions et densité des particules par rapport au milieu liquide, ainsi que vitesse souhaitée de ce milieu, de manière à ce que, dans un mode particulier de mise en oeuvre de l'invention, on puisse maintenir les particules en régime de lit fluidisé. Par exemple, dans le cas où les particules sont des agrégats cellulaires végétaux, le maillage peut être de 30 µm environ.

La taille des particules solides selon l'invention peut être choisie dans une large gamme. Elle est comprise en général entre 1 µm et 5 cm.

Pour des particules supérieures à 20 µm, c'est-à-dire retenues par des mailles de 20 µm, les grilles pourront, par exemple, être constituées par une toile notamment en inox, prise dans un cadre, ou d'un disque de matériau fritté tel que de l'inox fritté.

Pour des particules inférieures à 20 µm, on utilisera plutôt, à titre de grilles, des membranes de filtration de pores inférieures à la taille des particules.

La dimension des mailles ou pores des grilles ou membranes respectivement, sera, selon l'invention en général, de 0,5 µm à quelques millimètres, par exemple 5 mm.

Dans un mode de réalisation particulier, ladite paroi mobile, notamment la base du piston, incorpore un moyen de retenue des particules solides et de séparation du liquide, notamment de type grille telle que décrite ci-dessus.

De façon appropriée, dans le dispositif selon l'invention, l'enceinte peut être constituée d'une colonne cylindrique comportant une paroi cylindrique et deux parois d'extrémités formant les bases dudit cylindre, ces dernières comportant des grilles permettant l'entrée, la circulation et la sortie du milieu liquide de l'enceinte, mais retenant les particules à l'intérieur de l'enceinte, l'une au moins de ces deux grilles étant mobile.

Dans un mode particulier de réalisation du dispositif selon l'invention, l'enceinte comporte une ouverture obturable pratiquée dans sa paroi, permettant l'introduction de particules dans l'enceinte, ou encore l'introduction, ou le prélèvement, partielle ou totale du milieu contenant les particules solides. Le cas échéant, on préférera que ladite ouverture communique avec l'intérieur de l'enceinte uniquement lorsque l'enceinte atteint un certain volume, en particulier lorsque le volume de l'enceinte est maximum. Ainsi aucune perturbation ne risque de se produire au niveau de ladite ouverture au cours du processus de culture ou de réaction mis en oeuvre avec des volumes d'enceinte inférieurs.

En général, le dispositif selon l'invention comprend des moyens de circulation et traitement du liquide en dehors de l'enceinte, comportant notamment une canalisation, une pompe de préférence à débit variable et de préférence encore étant du type permettant d'inverser le sens du courant du milieu liquide, et éventuellement un récipient formant réservoir de milieu liquide, et des pinces d'isolement situées respectivement à l'entrée et à la sortie de l'enceinte et dudit récipient.

En particuler, ledit récipient peut comporter différents moyens pour contrôler, modifier et/ou homogénéiser la composition du milieu liquide, tels que par exemple des capteurs mesurant la température, le pH ou la concentration des gaz dissous, et des moyens de prélèvement du milieu ou d'addition de différentes substances ou compositions telles que milieu frais ou constituants de ce milieu, sous forme liquide, dissoute et/ou gazeuse.

Selon un mode de réalisation particulier, le dispositif comprend une canalisation supplémentaire, munie de préférence d'une pince d'isolement, permettant de réaliser une boucle de circulation du milieu liquide, ladite boucle incluant l'enceinte et la pompe, mais isolant le récipient précité formant réservoir.

Suivant un autre mode de réalisation particulier, le dispositif selon l'invention comporte en outre, notamment au niveau dudit récipient formant réservoir, des moyens permettant de soutirer, en alternance ou en continu, au moins une partie du milieu et de traiter celui-ci, notamment pour réaliser l'extraction de substances, en particulier celles initialement incorporées dans le milieu, ou celles produites lors de l'incubation du milieu avec lesdites particules, et permettant optionnellement de recycler à l'intérieur du dispositif, notamment dans le récipient formant réservoir, le milieu prélevé, après le traitement précité.

En particulier, les moyens précités comprennent avantageusement une ou plusieurs colonnes d'extraction, disposées en série ou en parallèle, contenant un support chromatographique approprié.

On comprend que, lorsque les particules constituées par exemple de cellules ou d'agrégats cellulaires, sont en suspension, on puisse réaliser leur agitation par le biais de la circulation du milieu liquide. De manière à éviter, dans ce cas, que des particules ne se regroupent dans une zone du réacteur et ne soient plus en mouvement dans le liquide, on évitera les formes pouvant engendrer des volumes morts.

La présente invention a également pour objet un procédé de culture de matériel biologique cellulaire sous forme de particules solides dans lequel on maintient sensiblement constante la densité volumique du dit matériel par rapport au volume de milieu de culture en contact avec les dites particules dans une chambre de culture, caractérisé en ce qu'on augmente le volume du milieu de culture compris dans la chambre de culture au fur et à mesure de l'augmentation du volume du dit matériel, en faisant varier le volume de la chambre de culture.

Dans un mode de réalisation du procédé, on met les dites particules solides du matériel biologique cellulaire en contact avec ledit milieu liquide au moyen d'un dispositif selon l'invention dans lequel l'enceinte est entièrement remplie de liquide, et l'on ajoute du milieu de culture liquide à l'intérieur de l'enceinte précitée, en augmentant le volume de ladite enceinte par déplacement de la dite paroi mobile, ce qui permet notamment de maîtriser à volonté la densité volumique desdites particules solides, par apport de milieu du culture en cours de culture.

Suivant un mode avantageux de mise en oeuvre du procédé de l'invention, lesdites particules solides sont maintenues en suspension, au moins par intermittence, dans le milieu liquide, notamment en régime de lit fluidisé, grâce à la circulation de ce milieu au travers de l'enceinte précitée.

De plus, dans un mode de réalisation du procédé de l'invention, on fait circuler le milieu liquide de façon continue ou intermittente à travers l'enceinte, éventuellement en inversant le sens de circulation régulièrement ou non, afin d'obtenir notamment une meilleure agitation, ainsi qu'une bonne répartition des particules en suspension dans l'enceinte et, si nécessaire, de décolmater les moyens de retenue précités, notamment les grilles.

Comme on l'a mentionné, lorsque le dispositif comporte à l'extérieur de l'enceinte des moyens de circulation comprenant des moyens de prélèvement du milieu et des moyens d'addition de milieu frais, on peut renouveler partiellement ou entièrement le milieu liquide en cours de réaction.

Selon l'invention, le contrôle et la mesure de tous les paramètres de la réaction (température, teneur en O₂, teneur en CO₂, évolution des teneurs des composés du milieu, etc...) se fait hors de l'enceinte et ce au cours de la réaction.

Suivant une autre caractéristique du procédé de l'invention, on peut réaliser en cours de réaction, en continu ou par intermittence, l'extraction de substances présentes dans le milieu liquide, soit qu'elles y avaient été initialement incorporées, soit qu'elles y ont été produites au cours de la réaction, grâce aux moyens décrits précédemment, permettant de soutirer, traiter et/ou recycler une partie du milieu.

Suivant un mode de réalisation particulier, les particules précitées sont constituées en totalité ou en partie de matière biologique cellulaire, l'autre partie pouvant être, dans le dernier cas, constituée d'une matière inerte, avantageusement une matière généralement utilisée par l'homme de l'art, notamment comme support ou matériau d'immobilisation, en particulier d'inclusion, du dit matériel biologique cellulaire.

La matière inerte, en particulier celle destinée à être utilisée pour immobiliser ladite matière vivante, peut être par exemple constituée d'une matrice de polymère, tel qu'un polyacrylamide, d'un gel ionotrope, tel qu'un gel de carraghénane, ou un gel d'alginate de calcium, d'une protéine à haut poids moléculaire, telle que du collagène sous forme de micro-éponge, ou de verre, de préférence sous forme de billes poreuses.

Le matériel biologique cellulaire précité peut être composé de cellules, telles que des cellules eucaryotes ou procaryotes, notamment des microorganismes tels que bactéries, moisissures, levures, euglènes, micro-algues, des cellules de plantes, des cellules animales, des agrégats cellulaires différenciés ou non, tels que des "TIL" (Tumour Infiltrating Lymphocytes), des embryons végétaux, ou des organes végétaux tels que des racines, tubercules, nodules organogènes, des plantules telles que des micropopagules ou des protocormes, en particulier protocormes d'orchidée.

En particulier, pour la mise en oeuvre du procédé de l'invention, on pourra utiliser des microorganismes de type floculant. Toutefois, lorsque la matière vivante est composée d'éléments de très petite taille, tels que des cellules isolées, des microorganismes, notamment des levures ou des bactéries, il est généralement préféré, pour la mise en oeuvre du procédé de l'invention, d'immobiliser d'une manière appropriée connue de l'homme de l'art, par exemple par inclusion, lesdits éléments au moyen d'une matière inerte telle que précédemment définie.

Le procédé de culture selon la présente invention peut être appliqué avantageusement à la production d'embryons somatiques de plante, en particulier de vigne ou de rosier, par culture d'agrégats de cellules embryogènes, de masses cellulaires pro-embryogènes, ou de pro-embryons, ou encore d'embryons déjà formés tels qu'embryons au stade dit globulaire, torpille ou cotylédonaire.

Un avantage de ce type de procédé, notamment appliqué à la culture d'un matériel biologique cellulaire tel que précédemment défini, en particulier cellules, agrégats cellulaires, embryons de végétaux, organes végétaux ou plantules, est de pouvoir s'affranchir de l'aération du milieu liquide directement dans la chambre de culture. En effet, des moyens d'aération tels que des cartouches d'oxygénation, peuvent être intégrés aux moyens de traitement du milieu de culture lors de la circulation à l'extérieur de la chambre de culture.

En ce qui concerne plus particulièrement les "TIL", on précise qu'au cours de leur préparation il y a un stade dans lequel ils se présentent sous forme de particules "pellets", obtenues après 7 jours de culture. Ainsi, au stade "pellets", les TIL peuvent être introduits dans le dispositif de l'invention, pour une production de masse. La possibilité qu'offre le dispositif de pouvoir fonctionner avec un très faible volume de milieu, surtout lorsque le récipient formant réservoir est mis hors-circuit, est ici particulièrement avantageuse car le milieu de culture est à un prix très élevé.

On peut appliquer le dispositif et le procédé selon l'invention à la transformation de molécules par des cellules végétales ou animales ou des microorganismes lorsque lesdistes molécules sont comprises dans le milieu liquide.

Par le procédé selon l'invention, on peut effectuer la production de molécules, telles que des protéines, des polyphénols tels que flavonoïdes, des saponines, des terpènes, des alcaloïdes, des stérols, des rétinoïdes ou des vitamines.

D'autres caractéristiques et avantages de la présente invention apparaîtront à la lumière de la description détaillée qui va suivre, donnée uniquement à titre d'illustration et qui ne saurait par conséquent limiter d'aucune façon la portée de l'invention.

La Figure 1 représente un schéma général d'un dispositif selon l'invention.

La Figure 2 représente un mode de réalisation des moyens d'extraction permettant l'extraction et la séparation de substances du milieu liquide.

La Figure 3 représente, en coupe, un mode de réalisation de l'enceinte du réacteur.

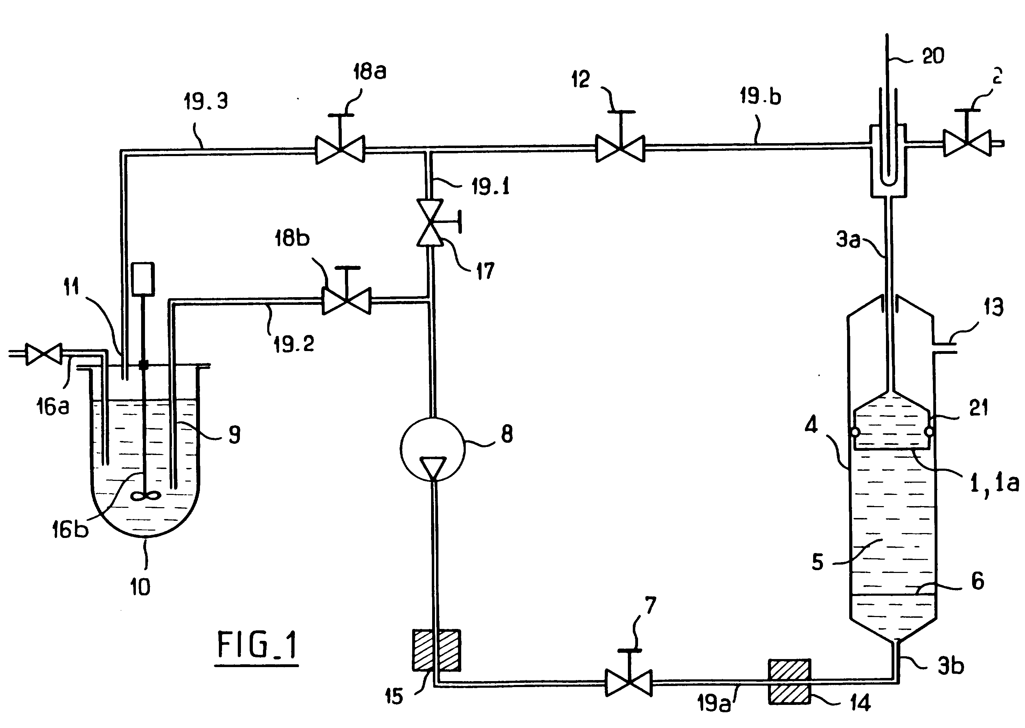

Un dispositif plus particulièrement adapté à la culture cellulaire, mais non exclusivement, est décrit sur les Figures 1 à 3. Dans le dispositif de la Figure 1 sont représentés en :
- 1 =: la paroi mobile de l'enceinte
- 1a =: grille de retenue des particules incorporée à la paroi mobile
- 2 =: un tuyau et pince d'isolement pour pompe de décolmatage
- 3a =: le moyen d'alimentation et évacuation du liquide de l'enceinte dans le piston de réglage du volume utile
- 3b =: le moyen d'alimentation et d'évacuation du liquide à l'extrémité inférieure de l'enceinte
- 4 =: la colonne cylindrique
- 5 =: l'enceinte à volume variable
- 6 =: une grille de retenue des particules
- 7 =: une pince d'isolement
- 8 =: une pompe de circulation à débit variable et/ou inversable
- 9 =: un tuyau de soutirage du milieu
- 10 =: un récipient formant réservoir de milieu liquide
- 11 =: un tuyau de retour de milieu
- 12 =: une pince d'isolement
- 13 =: une ouverture obturable notamment pour l'introduction de cellules dans l'enceinte
- 14 =: un filtre de stérilisation
- 15 =: une cartouche d'oxygénation
- 16a =: arrivées de milieu de culture frais, ou différents gaz par bullage, tels que O₂,CO₂, N₂
- 16b =: homogénéisateur de milieu
- 17, 18a, 18b =: des pinces d'isolement
- 19a, b, 19-1, 19-2, 19-3 =: les canalisations du circuit extérieur à l'enceinte
- 21 =: le piston de réglage du volume

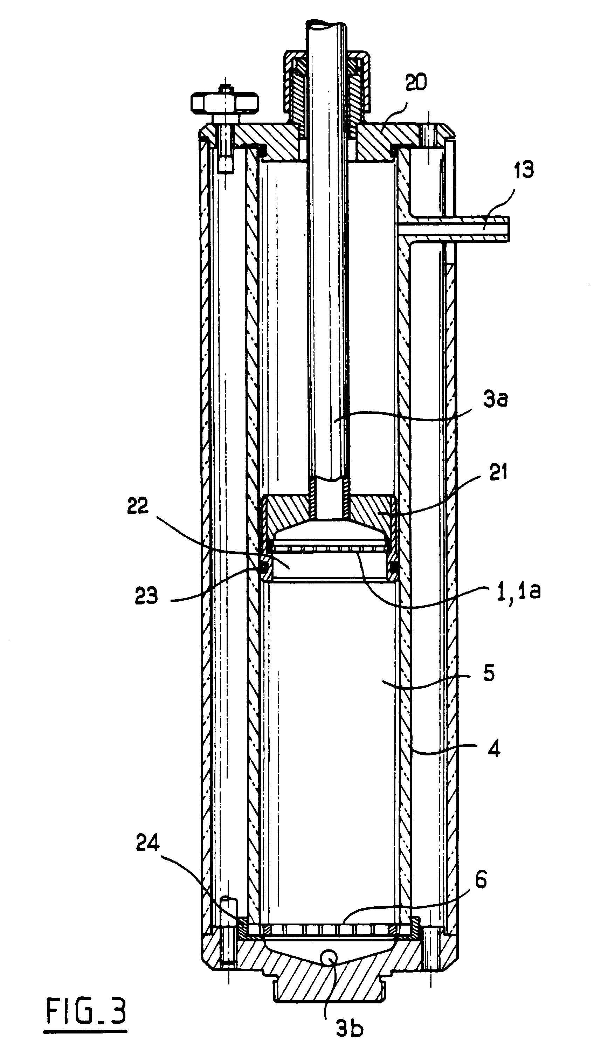

La Figure 1, la colonne cylindrique 4 constituant l'enceinte 5 à volume variable est terminée à chacune de ses extrémités par une forme conique. La paroi mobile 1 de l'enceinte est constituée par la base du piston 21. Le piston coopère de manière étanche avec l'intérieur de la colonne cylindrique 4 par un joint torique 23 (Figure 3). Le piston inclut les moyens 3a d'alimentation ou d'évacuation du liquide à l'extrémité supérieure de la colonne 4. A la base de la colonne, les moyens 3b permettent également l'alimentation ou l'évacuation du liquide. Les particules sont retenues dans l'enceinte par des grilles de maille 30µ, l'une (1a) incluse dans la base du piston 21, l'autre (6) à la base de la colonne.

Dans l'exemple représenté aux Figures 1 et 3, lorsque le piston est en position où l'enceinte a son volume maximum, l'enceinte communique avec une ouverture 13 permettant l'introduction ou le prélèvement de liquide ou l'introduction de particules dans l'enceinte.

Sur la Figure 1 sont représentés des moyens de circulation et traitement du liquide en dehors de l'enceinte, comportant une canalisation (19a, 19b, 19-1, 19-2, 19-3), une pompe péristaltique 8 à débit variable permettant de faire varier le débit du milieu liquide et/ou d'inverser le sens du courant. Tel que représenté sur la Figure 1, lorsque le liquide circule du haut vers le bas à l'intérieur de l'enceinte, il circule à l'extérieur par les canalisations (3b, 19a, 19-1, 19-b, 3a), les pinces 18a et 18b sont fermées, les pinces 7, 17 et 12 sont ouvertes. Lorsque le liquide circule dans l'autres sens, on peut faire circuler le liquide par les canalisations en dérivation 19-3 et 19-2, en passant par le récipient-réservoir 10, dans ce cas il faut ouvrir les pinces 18a et 18b et fermer la pince 17.

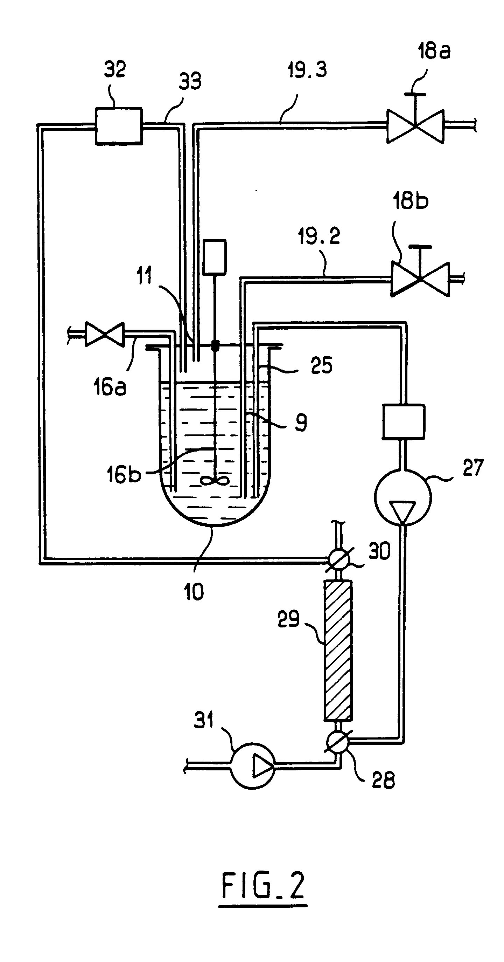

Sur la Figure 2 sont représentés les moyens conventionnels de soutirage, extraction et séparation de substances du milieu liquide à partir du récipient 10 :
- 25 =: un tuyau de soutirage pour extraction de composés du milieu
- 26 =: un filtre de stérilisation
- 27 =: une pompe de circulation à débit variable
- 28 =: un robinet à trois voies
- 29 =: une colonne de support chromatographique
- 30 =: un robinet à trois voies
- 31 =: une pompe de circulation à débit variable
- 32 =: un filtre de stérilisation
- 33 =: un retour de milieu après extraction de composés

Sur la Figure 2, on peut imaginer que la colonne de support chromatographique 29 soit remplacée par plusieurs colonnes différentes, en série ou en parallèle, pour permettre l'extraction alternative de composés différents.

Sur la Figure 3, qui montre un mode de réalisation d'une enceinte du réacteur adapté en particulier à la culture de cellules, d'agrégats cellulaires, d'embryons ou de racines, sont représentés en :
- 20 =: le couvercle de la colonne cylindrique
- 21 =: le corps du piston
- 22 =: une hotte cylindrique solidaire du piston
- 23 =: un joint torique
- 24 =: un joint plat.

Sur la Figure 3, le couvercle 20 de la colonne cylindrique 4 est amovible, ce qui peut permettre, après avoir dégagé le piston, de vidanger les particules solides telles que des racines, le cas échéant.

Sur la Figure 3, la base du cylindre 4 est disposée en butée sur la grille 6, elle-même venant en butée sur un joint plat 24 de façon étanche.

On décrit ci-après, à titre illustratif, un processus de régénération de production d'embryons végétaux, où les dispositif et procédé selon l'invention interviennent dans les trois étapes du processus suivantes :
- la multiplication de cellules embryogènes, d'agrégats de cellules embryogènes, de masses cellulaires pro-embryogènes, de pro-embryons et/ou d'embryons au stade globulaire,
- le développement en embryons d'agrégats de cellules embryogènes, de masses cellulaires pro-embryogènes ou de pro-embryons, et la maturation des embryons ainsi formés, et
- la phase de traitement des embryons pour induire leur germination en plantes.

Il est à noter que, pour des raisons de simplification, dans la présente description et les revendications ci-après, on désigne, par le terme "maturation" aussi bien le développement en embryons d'agrégats cellulaires, de masses pro-embryogènes ou de pro-embryons, que la maturation proprement dite des embryons ainsi formés, ces deux processus étant sucessifs ou plus ou moins simultanés en fonction des conditions de culture et/ou des espèces végétales considérées.

### 1) Remplissage de l'enceinte en milieu de culture

L'initiation de la maturation, telle que définie ci-dessus, se fait dans 80 ml de milieu de culture. Le piston est relevé dans la position correspondant à ce volume. On ferme les tuyaux 2 et 13.

La pompe péristaltique 8 raccordée au tuyau de soutirage 9 et au tuyau 19a permet le remplissage par le tuyau 3b au bas de la colonne, à partir du récipient 10. Lorsque l'enceinte est remplie, le milieu ressort par le tuyau 3a puis 19b par le haut. On branche le circuit sur le réservoir de milieu 10 en raccordant les tuyaux 19b et Il et on remplit l'enceinte et l'on autoclave le tout 20 minutes à 120°C. Le réservoir contient environ 11 de milieu de type Murashige et Skoog bien connu de l'homme de l'art, sans hormone de croissance.

### 2) Introduction des cellules sous forme d'agrégats

Après autoclavage, le dispositif est placé sous le flux d'une hotte afin de manipuler stérilement. On ouvre les tuyaux 2 et 13 et on relève le piston au maximum. On introduit 80 µl de cellules sous forme d'agrégats par ml de milieu par le tuyau 13 et on rince avec 1 ml de milieu stérile. On abaisse le piston au maximum pour éliminer l'air. Puis on ferme les tuyaux 2 et 13. On peut alors faire fonctionner la pompe 8 qui fera circuler le liquide dans la colonne 4, ce qui aura pour effet de mettre en suspension les fins agrégats (200 µm à 500 µm) dans les 80 ml de milieu de culture. Avantageusement, on fait fonctionner la pompe 8 en régime de débit variable, en faisant alterner des périodes de débit important et des périodes de débit faible, voire nul, ce qui a pour effet de maintenir une agitation au sein des agrégats en suspension. En variante, pour apporter le minimum de milieu, on peut relier directement 19a à 19b, par l'intermédiaire de la canalisation 19-1, et faire circuler en circuit fermé. On bénéficie ainsi généralement de l'effet positif de certains facteurs de croissance. De plus, dans ce cas, il est possible, et même avantageux, de faire fonctionner la pompe non seulement en régime variable comme décrit ci-dessus, mais aussi alternativement en sens inverse pour améliorer encore l'agitation au sein des agrégats.

### 3) Culture des embryons somatiques

Au bout de quatre jours de culture, le volume de la culture est augmenté en relevant le piston de quelques centimètres. On procède ainsi pendant 12 jours en augmentant chaque jour le volume de 30 ml environ pour atteindre un volume final de 450 à 500 ml (volume maximum de la chambre). On peut également, deux à trois fois au cours de la culture des embryons, renouveler complètement le milieu en vidant la colonne et le réservoir et en réalimentant le récipient 10 de milieu de culture frais par le tuyau 16a. On procède comme pour vider et arrêter le circuit, comme indiqué ci-après.

### 4) Arrêt de la culture des embryons somatiques

Après avoir ouvert le tuyau 2, on effectue le pompage du milieu contenu dans la colonne vers le réservoir avec la pompe 8 ; la chambre 5 se vide de milieu et les embryons se déposent sur le filtre 6 (grille) au bas de la colonne 4. On dévisse et soulève la colonne 4 (Figure 3) stérilement. On récupère le filtre 6 et les embryons qui se sont développés, et qui possèdent racines et cotylédons. Il est maintenant possible de les séparer et de les repiquer un par un sur un substrat approprié. **Analyse comparative:**

### 1) Première étape : multiplication des agrégats cellulaires.

Dans le système traditionnel de culture, l'ensemencement est réalisé à une densité de 20 µl de cellules par ml, dans 80 ml de milieu, dans un erlen de 250 ml. Après 15 jours de culture, la densité est de 80 microlitres par ml. A cette densité, le fractionnement en 4 erlens est nécessaire, sinon il se produit une nécrose des cellules. Cette opération est manuelle, et le risque de contamination microbienne est important.

Grâce au procédé du dispositif de l'invention, à l'aide du piston mobile, il est possible de ramener la densité à 20 µl d'agrégats cellulaires par ml de milieu en ajoutant automatiquement du milieu de culture sans risque de contamination exogène, ce qui est un atout indispensable pour une mise en oeuvre industrielle.

### 2) Deuxième étape = maturation et passage du stade agrégat cellulaire au stade embryon en forme de coeur.

Dans le système traditionnel, la densité de départ est de 1 à 2 µl de cellules par ml, et atteint 40 µl d'agrégats cellulaires par ml après 15 jours de culture (dans un erlen de 250 ml, contenant 80 ml de milieu). Or, il est nécessaire durant les 15 jours de maturation, de rester à une densité de 1 à 2 µl de cellules par ml, sinon il y a blocage du développement des cellules en embryons. L'apport manuel fréquent de milieu complémentaire n'est pas envisageable en application industrielle.

Par ailleurs, les cellules peuvent relarguer dans le milieu des inhibiteurs de type protéique et autres, qu'il est problématique d'éliminer de l'erlen.

Le procédé et dispositif de l'invention permettent de maintenir la densité volumétrique d'agrégats cellulaires comme on l'a vu et, en outre, le couplage d'un système de filtration approprié (25 à 33) sur les circuits de circulation extérieurs à la chambre de culture 5 permet d'éliminer périodiquement ou en continu les molécules du type inhibiteur. 3) Troisième étape : passage du stade coeur au stade embryon ayant une racine et deux cotylédons.

Pour avoir un développement correct des embryons, il est nécessaire, à cette étape, de remplacer le milieu de culture par un milieu différent, et ce, progressivement. Cette opération est techniquement problématique en erlens.

L'intérêt du procédé et dispositif de l'invention est là encore déterminant. De plus, pour la maturation et le traitement du milieu de culture, les procédés et dispositifs de l'invention permettent de contrôler et de mesurer les différents paramètres : O₂, CO₂, N₂, température, agitation, PH et composition du milieu de culture, sans risquer de détériorer ou blesser les embryons et les agrégats cellulaires.

## Revendications

1. Dispositif de culture de matériel biologique cellulaire sous forme de particules solides dans lequel on met en contact les dites particules solides avec un milieu de culture liquide, permettant de maintenir sensiblement constante la densité volumique du dit matériel biologique cellulaire par rapport au volume du dit milieu de culture, comportant :
- une chambre de culture constituée d'une enceinte (S) délimitée par des parois, dont au moins l'une des dites parois (1) peut être mobile de sorte que l'enceinte a un volume variable,
- des moyens (3a) et (3b) d'alimentation, d'évacuation et de circulation du liquide dans et de l'enceinte, et
- des moyens (1a, 6) de retenue des dites particules et de séparation d'avec le liquide pour maintenir celles-ci à l'ultérieur de l'enceinte,
caractérisé en ce qu'il comporte des moyens pour faire varier le volume du milieu de culture dans la chambre de culture, notamment pour faire augmenter le volume du milieu de culture au fur et à mesure du développement de la culture et de l'augmentation du volume de matériel biologique cellulaire, constitués par au moins l'une des dites parois (1) mobiles de l'enceinte, ladite paroi mobile comportant un moyen (1a) de retenue des particules solides et séparation d'avec le liquide.

2. Dispositif selon la revendication 1 caractérisé en ce que l'enceinte (5) définit, en partie au moins, un cylindre, la dite paroi mobile (1) de l'enceinte étant constituée par une base mobile du cylindre.

3. Dispositif selon l'une des revendications 1 ou 2, caractérisé en ce que l'enceinte (5) définit, en partie au moins, un cylindre (4) qui coopère de façon étanche avec un piston (21) lequel peut être déplacé à l'intérieur du cylindre de manière à faire varier le volume de l'enceinte en fonction de sa position, la base (1) du piston constituant une paroi mobile (1) de l'enceinte.

4. Dispositif selon la revendication 3, caractérisé en ce que l'enceinte est constituée d'une colonne cylindrique (4), les moyens d'alimentation et évacuation du liquide étant situés à chaque extrémité de celle-ci et l'une de ces extrémités étant constituée par le dit cylindre et le dit piston, le piston incluant un moyen (3a) d'alimentation ou d'évacuation du liquide étant situé à l'autre extrémité.

5. Dispositif selon l'une des revendications 1 à 4, caractérisé en ce que la base du piston (21), comporte un dit moyen (1a) de retenue des particules solides et séparation d'avec le liquide.

6. Dispositif selon l'une des revendications 1 à 5 caractérisé en ce que l'enceinte est constituée d'une colonne cylindrique comportant une paroi cylindrique et deux parois d'extrémités formant les bases du cylindre, ces dernières comportant des grilles (1a, 6) permettant la circulation du milieu liquide dans l'enceinte, mais retenant les particules à l'intérieur de l'enceinte, l'une au moins de ces deux grilles étant mobile.

7. Dispositif selon l'une des revendications 1 à 6 caractérisé en ce que l'enceinte comporte une ouverture (13) obturable pratiquée dans sa paroi, permettant l'introduction, ou le prélèvement, partielle ou totale du milieu, ou encore permettant l'introduction de particules dans l'enceinte.

8. Dispositif selon la revendication 7, caractérisé en ce que ladite ouverture (13) communique avec l'intérieur de l'enceinte uniquement lorsque le volume de celle-ci est maximum.

9. Dispositif selon l'une des revendications 1 à 8, caractérisé en ce que le dispositif comprend des moyens de circulation et traitement du liquide en dehors de l'enceinte, comportant une canalisation (19-2, 19-a, 19-b, 19-3), une pompe (8) de préférence à débit variable et de préférence encore étant du type permettant d'inverser le sens du courant du milieu liquide et éventuellement un récipient (10) formant réservoir de milieu liquide, et des pinces d'isolement (7, 12, 18a, 18b) situées respectivement à l'entrée et à la sortie de l'enceinte et dudit récipient (10).

10. Dispositif selon la revendication 9, caractérisé en ce que ledit récipient (10) comporte différents moyens (16b) pour contrôler, modifier et/ou homogénéiser la composition du milieu liquide, tels que par exemple des capteurs mesurant la température, le pH ou la concentration des gaz dissous, et des moyens (16a) de prélèvement du milieu ou d'addition de différentes substances ou compositions telles que milieu frais ou constituants de ce milieu, sous forme liquide, dissoute et/ou gazeuse.

11. Dispositif selon la revendication 9 ou 10, caractérisé en ce qu'il comprend une canalisation supplémentaire (19-1), munie de préférence d'une pince d'isolement (17), permettant de réaliser une boucle de circulation du milieu liquide, ladite boucle incluant l'enceinte (5) et la pompe (8), mais isolant le récipient (10) formant réservoir de milieu.

12. Dispositif selon l'une des revendications 1 à 11, caractérisé en ce qu'il comporte en outre, notamment au niveau du dit récipient (10) formant réservoir, des moyens (25 à 33) permettant de soutirer, en alternance ou en continu, au moins une partie du milieu et de traiter celui-ci, notamment pour réaliser l'extraction de substances, en particulier Celles initialement incorporées dans le milieu, ou celles produites lors de l'incubation du milieu avec les dites particules, et permettant optionnellement de recycler à l'intérieur du dispositif, notamment dans le récipient (10), le milieu prélevé, après le traitement précité.

13. Dispositif selon la revendication 12, caractérisé en ce que les moyens (25 à 33) précités comprennent une ou plusieurs colonne(s) d'extraction, disposée(s) en série ou en parallèle, contenant un support chromatographique approprié.

14. Procédé de culture de matériel biologique cellulaire sous forme de particules solides dans lequel on maintient sensiblement constante la densité volumique du dit matériel par rapport au volume de milieu de culture en contact avec les dites particules dans une chambre de culture, caractérisé en ce qu'on augmente le volume du milieu de culture compris dans la chambre de culture au fur et à mesure de l'augmentation du volume du dit matériel, en faisant varier le volume de la chambre de culture.

15. Procédé selon la revendication 14 dans lequel on met les particules solides en contact avec le dit milieu liquide au moyen d'un dispositif selon l'une des revendications 1 à 13, et dans lequel l'enceinte (5) est entièrement remplie de milieu de culture liquide et l'on ajoute du milieu de culture liquide à l'intérieur de l'enceinte en augmentant le volume de la dite enceinte par déplacement de la dite paroi mobile (21),et l'on maîtrise à volonté la densité volumique des dites particules solides, par apport de milieu de culture en cours de culture.

16. Procédé selon la revendication 14 ou 15, caractérisé en ce que les dites particules solides sont maintenues en suspension, au moins par intermittence, dans le milieu liquide, notamment en régime de lit fluidisé, grâce à la circulation de ce milieu au travers de l'enceinte (S).

17. Procédé selon l'une des revendications 14 à 16, caractérisé en ce qu'on fait circuler le milieu liquide de façon continue ou intermittente à travers l'enceinte (S), éventuellement en inversant le sens de circulation régulièrement ou non, afin d'obtenir notamment une meilleure agitation, ainsi qu'une bonne répartition, des particules en suspension dans l'enceinte (5), et, si nécessaire, de décolmater les moyens de retenue (1a, 6) précités, notamment les grilles.

18. Procédé selon l'une des revendications 14 à 17, caractérisé en ce que l'on renouvelle partiellement ou entièrement le milieu liquide en cours de réaction grâce aux moyens (16a) de prélèvement du milieu et d'addition de milieu frais.

19. Procédé selon l'une des revendications 14 à 18, caractérisé en ce que l'on réalise en cours de réaction, en continu ou par intermittence, l'extraction de substances présentes dans le milieu liquide, soit qu'elles y avaient été initialement incorporées, soit qu'elles y avaient été produites au cours de la réaction, grâce aux moyens (25 à 33) décrits précédemment, permettant de soutirer, traiter et/ou recycler une partie du milieu.

20. Procédé selon l'une des revendications 14 à 19, caractérisé en ce que les particules précitées sont constituées en totalité ou en partie de matériel biologique cellulaire, l'autre partie étant, dans ce dernier cas, constituée d'une matière inerte, notamment comme support ou matériau d'immobilisation, en particulier d'inclusion, du dit matériel biologique cellulaire.

21. Procédé selon l'une des revendications 14 à 20 caractérisé en ce que le dit matériel biologique cellulaire est composé de cellules, telles que des cellules eucaryotes ou procaryotes, notamment des microorganismes tels que bactéries, moisissures, levures, euglènes, micro-algues, des cellules de plantes, des cellules animales, des agrégats cellulaires différenciés ou non, tels que des "TIL" (Tumour Infiltrating Lymphocytes), des embryons végétaux, ou des organes végétaux tels que des racines, tubercules, nodules organogènes, des plantules telles que des micropopagules, les protocormes, en particulier protocormes d'orchidée.

22. Procédé selon l'une des revendications 14 à 20 appliqué à la transformation de molécules par des cellules animales ou végétales, ou de microorganismes, lorsque les dites molécules sont comprises dans le dit milieu liquide et que l'on cultive des dites cellules ou microorganismes.

23. Procédé selon la revendication 22 appliqué à la production de molécules, telles que des protéines, des polyphénols, des saponines, des terpènes, des alcaloïdes, des stérols, des rétinoïdes ou des vitamines.

24. Procédé selon l'une des revendications 14 à 20 appliqué à la production d'embryons somatiques de plante, en particulier de vigne ou de rosier, par culture d'agrégats de cellules embryogènes, de masses cellulaires pro-embryogènes ou de pro-embryons, ou encore d'embryons déjà formés tels qu'embryons au stade dit globulaire, torpille ou cotylédonaire.

## Claims

1. Device for culturing cellular biological material in the form of solid particles in which the said solid particles are brought into contact with a liquid culture medium, making it possible to keep the density by volume of the said cellular biological material substantially constant with respect to the volume of the said culture medium, containing:
- a culture chamber consisting of an enclosure (5) delimited by walls, of which at least one of the said walls (1) can be movable so that the enclosure has a variable volume,
- means (3a) and (3b) for supplying, discharging and circulating the liquid to and from the enclosure, and
- means (1a, 6) for retaining the said particles and for separating from the liquid in order to keep the particles within the enclosure,
characterized in that it contains means, consisting of at least one of the said movable walls (1) of the enclosure, for varying the volume of the culture medium in the culture chamber, especially in order to increase the volume of the culture medium during the development of the culture and the increase in the volume of the cellular biological material, the said movable wall containing a means (1a) for retaining the solid particles and separating from the liquid.

2. Device according to Claim 1, characterized in that the enclosure (5) defines, at least in part, a cylinder, the said movable wall (1) of the enclosure consisting of a movable base of the cylinder.

3. Device according to either of Claims 1 and 2, characterized in that the enclosure (5) defines, at least in part, a cylinder (4) which interacts in a leakproof way with a piston (21) which can be moved within the cylinder so as to vary the volume of the enclosure as a function of its position, the base (1) of the piston constituting a movable wall (1) of the enclosure.

4. Device according to Claim 3, characterized in that the enclosure consists of a cylindrical column (4), the means for supplying and discharging the liquid being situated at each end of the latter and one of these ends consisting of the said cylinder and the said piston, the piston including a means (3a) for supplying or discharging the liquid being situated at the other end.

5. Device according to one of Claims 1 to 4, characterized in that the base of the piston (21) contains a said means (la) for retaining the solid particles and separating from the liquid.

6. Device according to one of Claims 1 to 5, characterized in that the enclosure consists of a cylindrical column containing a cylindrical wall and two end walls forming the bases of the cylinder, the end walls containing screens (1a, 6) which make possible the circulation of the liquid medium in the enclosure but which retain the particles within the enclosure, at least one of these two screens being movable.

7. Device according to one of Claims 1 to 6, characterized in that the enclosure contains an opening (13), which can be closed up, made in its wall, which makes it possible to partially or completely introduce or withdraw the medium or alternatively which makes it possible to introduce particles into the enclosure.

8. Device according to Claim 7, characterized in that the said opening (13) communicates with the inside of the enclosure only when the volume of the latter is maximum.

9. Device according to one of Claims 1 to 8, characterized in that the device comprises means for circulating and treating the liquid outside the enclosure, containing a pipe (19-2, 19-a, 19-b, 19-3), a pump (8), preferably with a variable flow rate and again preferably being of the type which makes it possible to reverse the direction of the stream of the liquid medium, and optionally a receptacle (10) forming a reservoir for the liquid medium, and isolating clamps (7, 12, 18a, 18b) situated respectively at the inlet and at the outlet of the enclosure and of the said receptacle (10).

10. Device according to Claim 9, characterized in that the said receptacle (10) contains various means (16b) for controlling, modifying and/or homogenizing the composition of the liquid medium, such as, for example, sensors for measuring the temperature, the pH or the concentration of the dissolved gases, and means (16a) for withdrawing from the medium or for adding various substances or compositions such as fresh medium or constituents of this medium, in the liquid, dissolved and/or gaseous form.

11. Device according to Claim 9 or 10, characterized in that it comprises an additional pipe (19-1), preferably equipped with an isolating clamp (17), which makes it possible to produce a loop for circulating the liquid medium, the said loop including the enclosure (5) and the pump (8) but isolating the receptacle (10) which forms the reservoir of the medium.

12. Device according to one of Claims 1 to 11, characterized in that it additionally contains, especially at the said receptacle (10) forming the reservoir, means (25 to 33) which make it possible to draw off, alternately or continuously, at least part of the medium and to treat the latter, especially in order to extract substances, in particular those initially incorporated in the medium or those produced during the incubation of the medium with the said particles, and which make it possible, after the abovementioned treatment, optionally to recycle the withdrawn medium within the device, especially in the receptacle (10).

13. Device according to Claim 12, characterized in that the abovementioned means (25 to 33) comprise one or a number of extraction column(s), arranged in series or in parallel, containing an appropriate chromatographic substrate.

14. Process for culturing cellular biological material in the form of solid particles in which the density by volume of the said material with respect to the volume of the culture medium in contact with the said particles in a culture chamber is kept substantially constant, characterized in that the volume of the culture medium included in the culture chamber is increased during the increase in the volume of the said material, by varying the volume of the culture chamber.

15. Process according to Claim 14, in which the solid particles are brought into contact with the said liquid medium by means of a device according to one of Claims 1 to 13 and in which the enclosure (5) is entirely filled with liquid culture medium and liquid culture medium is added within the enclosure by increasing the volume- of the said enclosure by moving the said movable wall (21), and the density by volume of the said solid particles is controlled at will, by supplying culture medium during culturing.

16. Process according to Claim 14 or 15, characterized in that the said solid particles are kept suspended, at least intermittently, in the liquid medium, especially in the condition of a fluidized bed, by virtue of the circulation of this medium through the enclosure (5).

17. Process according to one of Claims 14 to 16, characterized in that the liquid medium is circulated continuously or intermittently through the enclosure (5), optionally by regularly or irregularly reversing the direction of circulation, in order especially to obtain better agitation, as well as good distribution of the suspended particles in the enclosure (5) and, if necessary, to clean the abovementioned retaining means (1a, 6), especially the screens.

18. Process according to one of Claims 14 to 17, characterized in that the liquid medium is partially or entirely renewed during reaction by virtue of the means (16a) for withdrawing the medium and for adding fresh medium.

19. Process according to one of Claims 14 to 18, characterized in that, during reaction, substances present in the liquid medium, whether they had been initially incorporated therein or whether they had been produced therein during the reaction, are continuously or intermittently extracted by virtue of the means (25 to 33) described above, which make it possible to draw off, treat and/or recycle part of the medium.

20. Process according to one of Claims 14 to 19, characterized in that the abovementioned particles consist entirely or partially of cellular biological material, the other part, in the latter case, consisting of an inert material, especially as substrate or material for immobilization, in particular for inclusion, of the said cellular biological material.

21. Process according to one of Claims 14 to 20, characterized in that the said cellular biological material is composed of cells, such as eukaryote or prokaryote cells, especially microorganisms such as bacteria, moulds, yeasts, euglenas or microalgae, plant cells, animal cells, differentiated or undifferentiated cell clusters, such as "TIL" (Tumour Infiltrating Lymphocytes), plant embryos or plant organs such as roots, tubers or organogenic nodules, or plantlets, such as micropropagules or protocorms, in particular orchid protocorms.

22. Process according to one of Claims 14 to 20, applied to the conversion of molecules by animal or plant cells or microorganisms when the said molecules are contained in the said liquid medium and when the said cells or microorganisms are cultured.

23. Process according to Claim 22, applied to the production of molecules, such as proteins, polyphenols, saponins, terpenes, alkaloids, sterols, retinoids or vitamins.

24. Process according to one of Claims 14 to 20, applied to the production of somatic plant embryos, in particular of the vine or rose, by culturing embryogenic cell clusters, proembryogenic cell masses or proembryos or alternatively embryos which have already formed, such as embryos at the so-called globular, torpedo or cotyledonary stage.

## Patentansprüche

1. Vorrichtung zur Kultivierung von biologischem Zellenmaterial in Form von festen Teilchen, in der man die genannten festen Teilchen mit einem flüssigen Kulturmedium in Kontakt bringt, und die es erlaubt, die Raumdichte des genannten biologischen Materials in bezug auf das Volumen des genannten Kulturmediums im wesentlichen konstant zu halten, die umfaßte
- eine Kultivierungskammer, die aus einem Behälter (5) besteht, der von Wänden begrenzt ist, wobei mindestens eine dieser Wände (1) beweglich sein kann, so daß der Behälter ein variables Volumen hat,
- Einrichtungen (3a) und (3b) zur Einführung, zum Abziehen und zur Zirkulation der Flüssigkeit zu, in und aus dem Behälter und
- Einrichtungen (1a, 6) zum Zurückhalten der genannten Teilchen und zur Abtrennung derselben mit der Flüssigkeit, um diese im Innern des Behälters zu halten,
dadurch gekennzeichnet, daß sie Einrichtungen aufweist, um das Volumen des Kulturmediums in der Kultivierungskammer variabel zu machen, insbesondere um das Volumen des Kulturmediums zu vergrößern entsprechend der Entwicklung der Kultur und der Zunahme des Volumens des biologischen Zellenmaterials, die bestehen aus mindestens einer der genannten beweglichen Wände (1) des Behälters, wobei die genannte bewegliche Wand eine Einrichtung (1a) zum Zurückhalten der festen Teilchen und zur Abtrennung derselben mit der Flüssigkeit aufweist.

2. Vorrichtung nach Anspruch 1, dadurch gekennzeichnet, daß der Behälter (5) mindestens zum Teil einen Zylinder darstellt, wobei die genannte bewegliche Wand (1) des Behälters eine bewegliche Basis des Zylinders darstellt.

3. Vorrichtung nach einem der Ansprüche 1 oder 2, dadurch gekennzeichnet, daß der Behälter (5) mindestens zum Teil einen Zylinder (4) darstellt, der über eine Dichtung (abgedichtet) mit einem Kolben (21) kooperiert, der im Innern des Zylinders verschoben werden kann, um das Volumen des Behälters als Funktion seiner Position zu variieren, wobei die Basis (1) des Kolbens eine bewegliche Wand (1) des Behälters darstellt.

4. Vorrichtung nach Anspruch 3, dadurch gekennzeichnet, daß der Behälter aus einer zylindrischen Säule (4) besteht, wobei die Einrichtungen zur Zuführung und zum Abziehen der Flüssigkeit an jedem Ende derselben angeordnet sind und eines dieser Enden von dem genannten Zylinder gebildet wird und der genannte Kolben, der eine Einrichtung (3a) zur Zuführung oder zum Abziehen der Flüssigkeit aufweist, an dem anderen Ende angeordnet ist.

5. Vorrichtung nach einem der Ansprüche 1 bis 4, dadurch gekennzeichnet, daß die Basis des Kolbens (21) eine genannte Einrichtung (1a) zum Zurückhalten der festen Teilchen und zur Abtrennung derselben mit der Flüssigkeit aufweist.

6. Vorrichtung nach einem der Ansprüche 1 bis 5, dadurch gekennzeichnet, daß der Behälter aus einer zylindrischen Säule besteht, die eine zylindrische Wand und zwei Stirnwände aufweist, welche die Basen des Zylinders bilden, wobei letztere Gitter (1a, 6) aufweisen, welche die Zirkulation des flüssigen Mediums in dem Behälter erlauben, jedoch die Teilchen im Innern des Behälters zurückhalten, wobei mindestens eines dieser beiden Gitter beweglich ist.

7. Vorrichtung nach einem der Ansprüche 1 bis 6, dadurch gekennzeichnet, daß der Behälter eine in seiner Wand angebrachte verschließbare Öffnung (13) aufweist, welche die Zuführung oder den Austrag eines Teils oder der Gesamtmenge des Mediums oder auch die Einführung von Teilchen in den Behälter erlaubt.

8. Vorrichtung nach Anspruch 7, dadurch gekennzeichnet, daß die genannte Öffnung (13) mit dem Innern des Behälters nur dann in Verbindung steht, wenn das Volumen desselben maximal ist.

9. Vorrichtung nach einem der Ansprüche 1 bis 8, dadurch gekennzeichnet, daß sie aufweist Einrichtungen zur Zirkulation und Behandlung der Flüssigkeit außerhalb des Behälters, die umfassen eine Rohrleitung (19-2, 19-a, 19-b, 19-3), eine Pumpe (8) mit vorzugsweise variablem Durchfluß, die vorzugsweise auch eine solche eines Typs ist, der die Umkehr der Fließrichtung des flüssigen Mediums erlaubt, und gegebenenfalls ein Gefäß (10), das ein Reservoir für das flüssige Medium darstellt, und Absperrklemmen (7, 12, 18a, 18b), die jeweils am Eingang und am Ausgang des Behälters und des Gefäßes (10) angeordnet sind.

10. Vorrichtung nach Anspruch 9, dadurch gekennzeichnet, daß das genannte Gefäß (10) verschiedene Einrichtungen (16b) zur Kontrolle, Modifizierung und/oder Homogenisierung der Zusammensetzung des flüssigen Mediums, beispielsweise Fühler zur Messung der Temperatur, des pH-Wertes oder der Konzentration der gelösten Gase, und Einrichtungen (16a) zur Entnahme des Mediums oder zur Zugabe verschiedener Substanzen oder Zusammensetzungen, wie frisches Medium oder Bestandteile dieses Mediums, in flüssiger, gelöster und/oder gasförmiger Form, aufweist.

11. Vorrichtung nach Anspruch 9 oder 10, dadurch gekennzeichnet, daß sie eine zusätzliche Rohrleitung (19-1) aufweist, die vorzugsweise mit einer Absperrklemme (17) ausgestattet ist, welche die Herstellung einer Zirkulationsschleife des flüssigen Mediums erlaubt, wobei die genannte Schleife den Behälter (5) und die Pumpe (8) enthält, das Gefäß (10), welches das Reservoir für das Medium bildet, jedoch isoliert.

12. Vorrichtung nach einem der Ansprüche 1 bis 11, dadurch gekennzeichnet, daß sie außerdem insbesondere im Bereich des genannten Gefäßes (10), das ein Reservoir bildet, Einrichtungen (25 bis 33) aufweist, die das alternierende oder kontinuierliche Abziehen mindestens eines Teils des Mediums und die Behandlung desselben, insbesondere zur Durchführung einer Extraktion von Substanzen, insbesondere solchen, die dem Medium anfänglich einverleibt worden waren, oder solchen, die während der Inkubation des Mediums mit den genannten Teilchen gebildet worden sind, erlauben, und die gegebenenfalls die Recyclisierung des entnommenen Mediums nach der obengenannten Behandlung ins Innere der Vorrichtung, insbesondere in das Gefäß (10), erlauben.

13. Vorrichtung nach Anspruch 12, dadurch gekennzeichnet, daß die genannten Einrichtungen (25 bis 33) eine oder mehrere Extraktionssäulen umfassen, die hintereinander oder parallel zueinander angeordnet sind und einen geeigneten chromatographischen Träger enthalten.

14. Verfahren zur Kultivierung von biologischem Zellenmaterial in Form von festen Teilchen, bei dem man die Raumdichte des genannten Materials in bezug auf das Volumen des Kulturmediums im Kontakt mit den genannten Teilchen in einer Kultivierungskammer im wesentlichen konstant hält, dadurch gekennzeichnet, daß man das Volumen des Kulturmediums, das in der Kultivierungskammer enthalten ist, entsprechend der Volumenzunahme des genannten Materials vergrößert, indem man das Volumen der Kultivierungskammer variiert.

15. Verfahren nach Anspruch 14, bei dem man die festen Teilchen unter Verwendung einer Vorrichtung gemäß einem der Ansprüche 1 bis 13 mit dem genannten flüssigen Medium in Kontakt bringt, und bei dem man den Behälter (5) vollständig mit dem flüssigen Kulturmedium füllt und dem Innern des Behälters flüssiges Kulturmedium zugibt, indem man das Volumen des genannten Behälters durch Verschiebung der beweglichen Wand (21) vergrößert, und daß man die Raumdichte der genannten festen Teilchen in bezug auf das Kulturmedium im Verlaufe der Kultivierung beliebig steuert (einstellt).

16. Verfahren nach Anspruch 14 oder 15, dadurch gekennzeichnet, daß man die genannten festen Teilchen mindestens intermittierend in dem flüssigen Medium in Suspension, insbesondere im Zustand eines fluidisierten Bettes, hält durch die Zirkulation dieses Mediums innerhalb des Behälters (5).

17. Verfahren nach einem der Ansprüche 14 bis 16, dadurch gekennzeichnet, daß man das flüssige Medium kontinuierlich oder intermittierend durch den Behälter (5) zirkulieren läßt, wobei man gegebenenfalls die Zirkulationsrichtung regelmäßig oder nicht-regelmäßig umkehrt, um insbesondere eine bessere Umrührung sowie eine gute Verteilung der in dem Behälter (5) suspendierten Teilchen zu erzielen und um erforderlichenfalls die obengenannten Rückhalteeinrichtungen (1a, 6), insbesondere die Gitter, zu spülen (zu reinigen).

18. Verfahren nach einem der Ansprüche 14 bis 17, dadurch gekennzeichnet, daß man das flüssige Medium im Verlaufe der Reaktion mittels der Einrichtungen (16a) zur Entnahme des Mediums und zur Zugabe von frischem Medium teilweise oder vollständig erneuert.

19. Verfahren nach einem der Ansprüche 14 bis 18, dadurch gekennzeichnet, daß man während des Verlaufs der Reaktion kontinuierlich oder intermittierend die in dem flüssigen Medium vorhandenen Substanzen sowohl diejenigen, die anfänglich zugegeben worden waren, als auch diejenigen, die im Verlaufe der Reaktion gebildet worden sind, mittels der obengenannten Einrichtungen (25 bis 33) extrahiert, die das Abziehen, Behandeln und/oder Rezirkulieren eines Teils des Mediums erlauben.

20. Verfahren nach einem der Ansprüche 14 bis 19, dadurch gekennzeichnet, daß die obengenannten Teilchen teilweise oder vollständig aus dem biologischen Zellenmaterial bestehen, wobei im ersteren Falle der übrige Teil aus einem inerten Material besteht, das insbesondere als Träger oder Immobilisierungsmaterial, insbesondere als Material zum Einschließen des genannten biologischen Zellenmaterials, fungiert.

21. Verfahren nach einem der Ansprüche 14 bis 20, dadurch gekennzeichnet, daß das genannte biologische Zellenmaterial besteht aus Zellen, wie Eucaryonten- oder Procaryontenzellen, insbesondere Mikroorganismen wie Bakterien, Schimmelpilzen, Hefen, Euglenen, Mikroalgen, Pflanzenzellen, Tierzellen, differenzierten oder nicht-differenzierten Zellaggregaten, wie "TIL" (Tumorinfiltrationslymphozyten), Pflanzenembryonen oder Pflanzenorganen, wie Wurzeln, Knollen, organbildenden Knospen, Keimlingen, wie Mikropopagules, Protocormes, insbesondere Orchideen-Protocormes.

22. Verfahren nach einem der Ansprüche 14 bis 20, das angewendet wird auf die Umwandlung von Molekülen durch Tierzellen oder Pflanzenzellen oder Mikroorganismen, wenn die genannten Moleküle in dem genannten flüssigen Medium enthalten sind, und daß man die genannten Zellen oder Mikroorganismen kultiviert.

23. Verfahren nach Anspruch 22, das angewendet wird auf die Herstellung von Molekülen, wie Proteinen, Polyphenolen, Saponinen, Terpenen, Alkaloiden, Sterinen, Retinoiden oder Vitaminen.

24. Verfahren nach einem der Ansprüche 14 bis 20, das angewendet wird auf die Herstellung von somatischen Pflanzenembryonen, insbesondere solchen der Weinrebe oder des Rosenstockes, durch Aggregat-Kultivierung von embryogenen Zellen, proembryogenen oder Proembryonen-Zellmassen oder auch bereits gebildeten Embryonen, wie Embryonen im sogenannten GlobularTorpillen- oder Cotyledonar-Stadium.
